# EUROPEAN PATENT SPECIFICATION

(11) **EP 1 428 811 B1**
(45) Date of publication and mention of the grant of the patent: **20.04.2011**
(21) Application number: 03078647.9
(22) Date of filing: 18.02.1998
(51) Int. Cl.: C07C 19/08, C07C 19/10, C07C 17/07, C07C 17/383, C07C 41/22, C07C 43/12

(54) **Azeotropic compositions comprising 1,1,1,2,3,3,3-heptafluoropropane and processes using said compositions**
Azeotropische Zusammensetzungen enthaltend 1,1,1,2,3,3,3-Heptafluorpropan und Verfahren, die diese Zusammensetzungen verwenden
Compositions azeotropiques contenant 1,1,1,2,3,3,3-heptafluoropropane et procédés utilisant ces compositions

(30) Priority: 19.02.1997 US 38430 P; 16.06.1997 US 49723 P; 25.08.1997 US 56795 P
(43) Date of publication of application: 16.06.2004
(62) Divisional of application: 98905114.9
(73) Proprietor: E.I. DU PONT DE NEMOURS AND COMPANY, Wilmington, DE 19898 (US)
(72) Inventor: Rao, V.N.Mallikarjuna, Wilmington, DE 19809 (US); Sievert, Allen Capron, Elkton, MD 21921 (US)
(74) Representative: Towler, Philip Dean

(56) References cited:
- EP-A- 0 442 075
- EP-A- 0 634 384
- EP-A- 0 676 386
- WO-A-95/04022
- WO-A-95/32935

## Description

### FIELD OF THE INVENTION

This invention relates to azeotropic compositions comprising CF₃CHFCF₃ and HF, and to a process for recovering HF from a product mixture comprising CF₃CHFCF₃ and HF.

### BACKGROUND

A number of chlorine-containing halocarbons are considered to be detrimental toward the Earth's ozone layer. There is a world-wide effort to develop materials having lower ozone depletion potential that can serve as effective replacements. For example, the hydrofluorocarbon, 1,1,1,2-tetrafluoroethane (HFC-134a) is being used as a replacement for dichlorodifluoromethane (CFC-12) in refrigeration systems. The production of hydrofluorocarbons (i.e., compounds containing only carbon, hydrogen and fluorine), has been the subject of considerable interest to provide environmentally desirable products for use as solvents, blowing agents, refrigerants, cleaning agents, aerosol propellants, heat transfer media, dielectrics, fire extinguishants and power cycle working fluids (see, e.g., PCT International Publication No. WO 93/02150).

EP 0634384 A1 relates to a process for producing CF₃CHFCF₃ wherein CF₃CF=CF₂ is reacted with HF in the presence of a weakly basic ion exchanger whose reactive centres comprise tertiary amino groups. The CF₃CHFCF₃ product is isolated by condensing the gas obtained from the reaction. Entrained traces of HF from the crude product may be removed by by means of scrubbing processes.

### SUMMARY OF THE INVENTION

Azeotropic compositions (e.g., an azeotropic composition consisting essentially of from about 29.9 to about 41.3 mole percent HF and from about 70.1 to 58.7 mole percent CF₃CHFCF₃) are provided which comprise CF₃CHFCF₃ and HF, wherein said HF is present in an amount effective to form an azeotropic combination with said CF₃CHFCF₃.

The present invention further provides a process for recovering HF from a product mixture comprising HF and CF₃CHFCF₃. The process comprises (1) distilling the product mixture to remove all products which have a lower boiling point than the lowest boiling azeotrope containing HF and CF₃CHFCF₃; and (2) distilling said azeotrope to recover HF as an azeotropic composition containing HF and CF₃CHFCF₃.

### DETAILED DESCRIPTION

The hydrodehalogenation of CF₃CCl₂CF₃ (CFC-216aa) may be effected in a manner which removes a single fluorine from an end carbon while removing at least one chlorine from the internal carbon to produce CF₃CCl=CF₂ (CFC-1215xc) and CF₃CH=CF₂ (HCFC-1225zc). This hydrodehalogenation generally produces a product comprising CF₃CCl=CF₂, CF₃CH=CF₂, HF and HCl, and involves the use of advantageously catalytic components employing copper, nickel and/or chromium. Suitable components include halides such as CuF, CuCl, CuCl₂, CuClF, NiF₂, NiCl₂, NiClF, CrF₃, CrCl₃, CrCl₂F and CrClF₂; oxides such as CuO, NiO, and Cr₂O₃; and oxyhalides such as copper oxyfluoride and chromium oxyfluoride. Oxyhalides may be produced by conventional procedures such as, for example, halogenation of metal oxides.

The catalysts may contain other components, some of which are considered to improve the activity and/or longevity of the catalyst composition. Preferred catalysts include catalysts which are promoted with compounds of molybdenum, vanadium, tungsten, silver, iron, potassium, cesium, rubidium, barium or combinations thereof. Also of note are chromium-containing catalysts which further contain zinc and/or aluminum or which comprise copper chromite.

The catalyst may be supported or unsupported. Supports such as metal fluorides, alumina and titania may be advantageously used. Particularly preferred are supports of fluorides of metals of Group IIB, especially calcium. A preferred catalyst consists essentially of copper, nickel and chromium oxides (each of said oxides being preferably present in equimolar quantities) preferably promoted with potassium salt, on calcium fluoride.

An especially preferred catalyst contains proportionally about 1.0 mole CuO, about 0.2 to 1.0 mole NiO, about I to 1.2 moles Cr₂O₃ on about 1.3 to 2.7 moles CaF₂, promoted with about 1 to 20 weight%, based on the total catalyst weight, of an alkali metal selected from K, Cs, and Rb (preferably K). When K is the promoter, the preferred amount is from about 2 to 15 weight% of the total catalyst.

This catalyst can be prepared by coprecipitating, from an aqueous medium, salts of copper, nickel and chromium (and optionally aluminum and zinc), with and preferably on calcium fluoride; washing, heating and drying the precipitate. An alkali metal compound (e.g., KOH, KF or K₂CO₃) is then deposited on the dried precipitate, followed by calcination to convert the copper, nickel and chromium to the respective oxides. Any soluble copper, nickel and chromium compound may be used, but the chlorides and nitrates are preferred, with the nitrates being especially preferred. Alternatively, promoters such as KOH, KF and K₂CO₃ may be added prior to co-precipitation.

Another group of catalysts which may be used for the conversion of CF₃CCl₂CF₃ contains proportionally about 1.0 mole CuO, about 0.2 to 1.0 mole NiO, about 1 to 1.2 moles Cr₂O₃, about 0.4 to 1.0 mole MoO₃, and about 0.8 to 4.0 mole CaF₂, optionally promoted with at least one compound from the group consisting of MgF₂, MnF₂, and BaF₂. Palladium or WO₃ may also be present.

The catalyst may be granulated, pressed into pellets, or shaped into other desirable forms. The catalyst may contain additives such as binders and lubricants to help insure the physical integrity of the catalyst during granulating or shaping the catalyst into the desired form. Suitable additives include carbon and graphite. When binders and/or lubricants are added to the catalyst, they normally comprise about 0.1 to 5 weight percent of the weight of the catalyst.

The catalyst may be activated prior to use by treatment with hydrogen, air, or oxygen at elevated temperatures. After use for a period of time in the process of this invention, the activity of the catalyst may decrease. When this occurs, the catalyst may be reactivated by treating it with hydrogen, air or oxygen, at elevated temperature in the absence of organic materials.

The molar ratio of hydrogen to CF₃CCl₂CF₃ fed to the process typically ranges from about 1:1 to about 30:1, and is preferably at least about 3:1.

The hydrodehalogenation process of CF₃CCl₂CF₃ is suitably conducted at a temperature in the range of from about 300°C to 450°C, preferably from about 350°C to about 400°C. The contact time of reactants with the catalyst bed (i.e., the volume of the catalyst bed divided by the volumetric flow rate at the temperature and pressure of the reaction) is typically from about 5 seconds to about 4 minutes:

The product from the hydrodehalogenation reaction of CF₃CCl₂CF₃ comprises CF₃CH=CF₂, CF₃CCl=CF₂, HCl and HF and typically other compounds such as unreacted CF₃CCl₂CF₃ and partially reacted compounds such as CF₃CHClCF₃. These products may be separated by conventional means such as distillation and/or decantation and the components may be used individually. For example, CF₃CH=CF₂ (HFC-1225zc) may be used as a co-monomer for producing fluorine-containing polymers. Unreacted starting material (CFC-216aa) can be recycled to the hydrodehalogenation reactor.

CF₃CCl=CF₂ (CFC-1215xc) may be reacted with HF to produce CF₃CHFCF₃ (HFC-227ea), which is a useful fire extinguishant. The reaction is typically conducted at an elevated temperature in either the liquid or vapor phase using a fluorination catalyst. For example, CF₃CCl=CF₂ may be reacted with HF in the liquid phase at a temperature of from about 100 to 175°C over a pentavalent antimony catalyst (e.g., SbF₅) to produce CF₃CHFCF₃; or CF₃CCl=CF₂ may be reacted with HF in the vapor phase at a temperature of from about 300 to 400°C over an unsupported or supported trivalent chromium catalyst (e.g., Cr₂O₃ or Cr₂O₃/AlF₃). Preferably, the mole ratio of HF to CF₃CCl=CF₂ used for CF₃CHFCF₃ production is at least about 5:1.

The reaction products from the hydrofluorination may be separated by conventional techniques, such as distillation. CF₃CHFCF₃ may form azeotropic combinations with HF and/or HCl; and conventional decantation/distillation may be employed if further purification of CF₃CHFCF₃ is desired.

An azeotrope is a liquid mixture that exhibits a maximum or minimum boiling point relative to the boiling points of surrounding mixture compositions. A characteristic of minimum boiling azeotropes is that the bulk liquid composition is the same as the vapor compositions in equilibrium therewith, and distillation is ineffective as a separation technique. It has been found, for example, that CF₃CHFCF₃ (HFC-227ea) and HF form a minimum boiling azeotrope. This azeotrope can be produced as a co-product with HFC-227ea. As discussed further below, compositions may be formed which consist essentially of azeotropic combinations of hydrogen fluoride with HFC-227ea. These include a composition consisting essentially of from about 29.9 to 41.3 mole percent HF and from about 70.1 to 58.7 mole percent HFC-227ea (which forms an azeotrope boiling at a temperature between about -25°C and about 100°C at a pressure between about 77 kPa and about 3764 kPa). HFC-227ea can be separated from the HF in such azeotropes by conventional means such as neutralization and decantation. Azeotropic compositions of hydrofluorocarbons and HF (e.g.. an azeotrope recovered by distillation of fluorination reactor effluent) are useful as recycle to the fluorination reactor, where the recycled HF can function as a reactant and the recycled hydrofluorocarbon can function to moderate the temperature effect of the heat of reaction. Thus, for example, the process for producing CF₃CHFCF₃ can further comprise the steps of recovering a portion of the CF₃CHFCF₃ as an azeotropic composition of CF₃CHFCF₃ and HF and recycling said azeotropic composition to the reactor.

Reference is made to U.S. Patent No. 5,563,304 for a discussion of vapor phase hydrofluorination.

Pressure is not critical for the hydrofluorination, hydrogenation, and hydrodehalogenation processes described above. Atmospheric and superatmospheric pressures (e.g., pressure from about 100 kPa to 7000 kPa) are the most convenient and are therefore preferred.

The hydrofluorination, hydrogenation and hydrodehalogenation reactions may be conducted in any suitable reactor. The reaction vessel should be constructed from materials which are resistant to the corrosive effects of hydrogen fluoride such as Inconel^{™} nickel alloy and Hastelloy^{™} nickel alloy.

The CF₃CCl₂CF₃ used as a reactant may be produced by known art methods such as disclosed in U.S. Patent No. 5,057,634.

### HFC-227ea/HF Azeotrope

As noted above, the present invention provides a composition which consists essentially of hydrogen fluoride and an effective amount of CF₃CHFCF₃ to form an azeotropic composition with hydrogen fluoride. By effective amount is meant an amount which, when combined with HF, results in the formation of an azeotrope or azeotrope-like mixture. As recognized in the art, an azeotrope or an azeotrope-like composition is an admixture of two or more different components which, when in liquid form under given pressure, will boil at a substantially constant temperature, which temperature may be higher or lower than the boiling temperatures of the components, and which will provide a vapor composition essentially identical to the liquid composition undergoing boiling.

For the purpose of this discussion, azeotrope-like compositions means a composition that behaves like an azeotrope (i.e., has constant-boiling characteristics or a tendency not to fractionate upon boiling or evaporation). Thus, the composition of the vapor formed during boiling or evaporation is the same as or substantially the same as the original liquid composition. Hence, during boiling or evaporation, the liquid composition, if it changes at all, changes only to a minimal or negligible extent. This is to be contrasted with non-azeotrope-like compositions in which during boiling or evaporation, the liquid composition changes to a substantial degree.

Accordingly, the essential features of an azeotrope or an azeotrope-like composition are that at a given pressure, the boiling point of the liquid composition is fixed and that the composition of the vapor above the boiling composition is essentially that of the boiling liquid composition (i.e., no fractionation of the components of the liquid composition takes place). It is also recognized in the art that both the boiling point and the weight percentages of each component of the azeotropic composition may change when the azeotrope or azeotrope-like liquid composition is subjected to boiling at different pressures. Thus, an azeotrope or an azeotrope-like composition may be defined in terms of the unique relationship that exists among components or in terms of the compositional ranges of the components or in terms of exact weight percentages of each component of the composition characterized by a fixed boiling point at a specified pressure It is also recognized in the art that various azeotropic compositions (including their boiling points at particular pressures) may be calculated (see, e.g., W. Schotte Ind. Eng. Chem. Process Des. Dev. (1980) 19, 432-439). Experimental identification of azeotropic compositions involving the same components may be used to confirm the accuracy of such calculations and/or to modify the calculations at the same or other temperatures and pressures.

It has been found that azeotropes of HFC-227ea and HF are formed at a variety of temperatures and pressures. Between 78 kPa (at a temperature of -25°C) and 3764 kPa (at a temperature of 100°C) azeotropic compositions consisting essentially of HFC-227ea and HF range from about 29.9 mole percent HF (and 70.1 mole percent HFC-227ea) to about 41.3 mole percent HF (and 58.7 mole percent HFC-227ea). An azeotrope of HF and CF₃CHFCF₃ has been found at -10°C and 21.9 psia (151 kPa) consisting essentially of about 40.9 mole percent HF and about 59.1 mole percent HFC-227ea). An azeotrope of HF and CF₃CHFCF₃ has also been found at 70°C and 261.2 psia (1800 kPa) consisting essentially of about 37.0 mole percent HF and about 63.0 mole percent HFC-227ea. Based upon the above findings, it has been calculated that an azeotropic composition of about 41.3 mole percent HF and 58.7 mole percent HFC-227ea can be formed at -25°C and 78 kPa and an azeotropic composition of about 29.9 mole percent HF and 70.1 mole percent HFC-227ea can be formed at 125°C and 3764 kPa. Accordingly, the present invention provides an azeotrope or azeotrope-like composition consisting essentially of from about 29.9 to about 41.3 mole percent HF and from about 70.1 to 58.7 mole percent HFC-227ea, said composition having a boiling point from about -25°C to 78 kPa to about 100°C at 3764 kPa.

Processes may employ azeotropic distillation of HF with CF₃CHFCF₃. Product mixtures obtained from a variety of sources can be distilled. These sources include product mixtures produced by fluorination with HF of CF₃CF=CF₂ to afford HFC-227ea/HF. The described catalytic fluorination with HF reactions can be done in either the liquid or vapor phase using procedures known in the art. The product mixture may be distilled to remove all products which have a lower boiling point than the lowest boiling azeotrope containing HF and HFC-227ea. Such low-boiling materials can include, for example, HCl. For continuous processes, distillate and azeotropes with higher boiling points can be advantageously removed from appropriate sections of the distillation column. The lowest boiling azeotrope containing HF and CF₃CHFCF₃ may then be distilled such that HF is recovered as an azeotropic composition containing HF together with CF₃CHFCF3.

Where the mixture (after distilling components boiling at lower temperatures than the lowest boiling azeotrope of HF with CF₃CHFCF₃) consists essentially of HF and CF₃CHFCF₃, HF may be recovered as an azeotrope consisting essentially of CF₃CHFCF₃ and HF. If excess amounts of CF₃CHFCF₃ or HF remain after azeotropes are recovered from these mixtures, such excess may be recovered as a relatively pure compound. The distillation of azeotropes containing HF and CF₃CHFCF₃ may be done at a wide variety of temperatures and pressures. Typically the temperature is between about -25°C and about 125°C and the pressure is between 78 kPa and 3764 kPa. The process of this invention includes embodiments where azeotropic compositions containing from about 58.7 to about 70.1 mole percent CF₃CHFCF₃ are recovered. HF may be recovered for example, from a product mixture including CF₃CHFCF₃ formed by the reaction of C_{F}3CF=C_{F}2 with HF.

The HFC-227ea/HF azeotrope can be used as an HF source to fluorinate numerous compounds. Optionally, such fluorinations can employ a fluorination catalyst. The fluorinations can be done in the liquid phase using typical catalysts such as SbCl₅. The fluorinations can also be done in the vapor phase using typical catalysts such as Cr₂O₃. The following compounds, either individually or in mixed blends, can be fluorinated with the HF azeotrope to provide a variety of compositions wherein the ratio of the fluorination product(s) to CF₃CHFCF₃ is about 1:99, or more (depending upon the azeotropic combination of CF₃CHFCF₃ and HF used, and the degree of fluorination).

By fluorination precursors to the component (c2) compound(s) is meant compounds which react with HF (optionally in the presence of a fluorination catalyst) to produce the corresponding component (c2) compound(s). Fluorination precursors include saturated compounds having the formula

CₙH_{2n+2-a-b}Clₐ₊ₓF_{b-x}O_{c}

wherein x is an integer from 1 to b. Examples of saturated precursors and corresponding products are as follows:

| SATURATED PRECURSOR | PRODUCT |
|---|---|
| CH₂Cl₂ | CH₂F₂ |
| CHCl₂CHCl₂ | CHF₂CHF₂ |
| CF₃CH₂Cl | CF₃CH₂F |
| CH₂ClCF₂CHF₂ | CH₂FCF₂CHF₂ |
| CH₃CF₂CCl₃ | CH₃CF₂CF₃ |
| CHCl₂CH₂CCl₃ | CHF₂CH₂CF₃ |
| CHCl₂OCF₂CHF₂ | CHF₂OCF₂CHF₂ |
| CF₃CHClOCHF₂ | CF₃CHFOCHF₂ |
| CHF₂OCHCl₂ | CHF₂OCHF₂ |
| CClF₂OCHF₂ | CF₃OCHF |

Fluorination precursors also include unsaturated compounds having the formula

CₙH_{2n+1-a-b}Cl_{a+y}F_{b-y-1}O_{c}

wherein y is an integer from 0 to b-1. Examples of unsaturated precursors and corresponding products are as follows:

| UNSATURATED PRECURSOR | PRODUCT |
|---|---|
| CH₂=CF₂ | CH₃CF₃ |
| CH₂=CH₂ | CH₃CH₂F |
| CH₂=CCl₂ | CH₃CCl₂F |
| CF₃CH=CH₂ | CF₃CH₂CH₂F |
| CF₃CCl=CCl₂ | CF₃CHClCClF₂ |
| CF₃CF=CHF | CF₃CHFCHF₂ |
| CF₃CH=CF₂ | CF₃CH₂CF₃ |
| CF₃OCF=CF₂ | CF₃OCHFCF₃ |

Of particular note are processes where for component (b) a is 0 and b is 2n+1, or less.

These fluorinations include processes for producing compositions wherein the molar ratio of component (c2) to CF₃CHFCF₃ is between about 1:99 and about 41.3:58.7. This process comprises (A) combining (i) an azeotrope or azeotrope-like composition consisting essentially of CF₃CHFCF₃ and HF wherein the ratio of HF to the (c1) component is at least equal to the desired ratio of component (c2) to the respective component (c1) compound with the precursor component (ii).

Of note are embodiments of this fluorination where the CF₃CHFCF₃/HF, azeotrope combined with the precursor(s) is obtained by (1) distilling a product mixture comprising HF and a compound selected from the group consisting of CF₃CHFCF₃, CF₃CH₂CF₃ and CHF₂CH₂CF₃ to remove all products which have a lower boiling point than the lowest boiling azeotrope containing HF and said compound; and (2) distilling said azeotrope to recover HF as an azeotropic composition containing HF and said compound. Also of note are processes where the fluorination precursors include precursors for at least two saturated compounds of the formula CₙH_{2n+2-a-b}ClₐF_{b}O_{c}, where c is 1 for at least one of said saturated compounds.

The fluorination product components containing component (c1) and component (c2) may be separated by conventional means such as distillation, selective sorption and/or decantation. The compositions of this invention comprising components (c1) and (c2) (including at least one ether) are useful, for example, as aerosol propellants, fire extinguishants, and/or refrigerants. Some of the compounds of the component (c1)/component (c2) combinations may form HCl azeotropes. The HCl can be separated from those combinations by extractive distillation or sorption on activated carbon. A number of the combinations may boil too close together to separate by distillation, forming zeotropic blends (i.e., blends boiling within a limited temperature range). Some of the combinations may form binary or even ternary azeotropes. The azeotropes, zeotropes and individual compounds can be collected from different parts of a distillation column.

Without further elaboration, it is believed that one skilled in the art can, using the description herein, utilize the present invention to its fullest extent. The following specific embodiments are to be construed as illustrative, and not as constraining the remainder of the disclosure in any way whatsoever.

### REFERENCE EXAMPLES

### Hydrodehalogenation Catalyst Preparation for the Examples

Aqueous calcium nitrate (2.7 moles) is mixed with aqueous potassium fluoride (5.4 moles), heated and stirred briefly at 100°C to form a slurry of CaF₂. To this slurry is added copper nitrate (1 mole), nickel nitrate (1 mole) and chromium nitrate (1 mole) as solids. The slurry is stirred at 70 to 80°C until the salts, other than CaF₂, dissolve. This is followed by adding 0.1 mole of aqueous potassium hydroxide over 1 hour and boiling the mixture briefly. The slurry is cooled to 40 to 50°C and filtered. The solid is washed exhaustively to reduce the potassium content to an undetectable level. After drying, potassium hydroxide is added as a solution in quantities sufficient to provide a catalyst containing 9 weight % potassium. After drying again, the catalyst is calcined at 600°C for 8 to 16 hours, then granulated and screened to I to 2 mm particles. The catalyst is mixed with 1 to 5 wt% "Sterotex" powdered lubricant (registered trademark of Capital City Products Co., Columbus Ohio, division of Stokely-Van Camp, for its edible hydrogenated vegetable oil) to give 1/8" x 1/8" (3.2 mm x 3.2 mm) cylindrical pellets from a Stokes tablet machine.

### General Procedure for Product Analysis for the Examples

The products leaving the reactor were analyzed on line using a gas chromatograph. The column consisted of a 20' (6.1 m) x 1/8" (3.2 mm) stainless steel tube containing Krytox^{™} perfluorinated polyether on an inert support. Helium was used as the carrier gas. The product analyses are reported in mole%.

### Legend:

| | |
|---|---|
| 216aa is CF₃CCl₂CF₃ | 226da is CF₃CHClCF₃ |
| 236fa is CF₃CH₂CF₃ | 245fa is CF₃CH₂CHF₂ |
| 1225zc is CF₃CH=CF₂ | CT is contact time |
| 1215xc is CF₃CCl=CF₂ | conv. is conversion sel. is selectivity |

### REFERENCE EXAMPLE 1

A 15" (381 mm) X 1/4" (6.4 mm) O.D. Inconel^{™} 600 nickel alloy U-tube reactor used for hydrodehalogenation (HDH) was charged with catalyst (21.7 g, 18 mL) pellets prepared substantially in accordance with the Catalyst Preparation described above. The HDH catalyst was in use for a variety of runs totaling 646 hours prior to its use in Example 1. Before starting the runs shown in Example 1, the HDH catalyst was regenerated with 50 sccm (8.3 x 10⁻⁷ m³/s) 50% air/50% N₂ at 350°C for 1 hour; 50 sccm (8.3 x 10⁻⁷ m³/s) air at 400°C for 2 hours; followed by purging with 200 sccm (3.3 x 10⁻⁶ m³/s) N₂ at 400°C for 40 minutes; and finally reduced with 50 sccm (8.3 x 10⁻⁷ m³/s) H₂ at 300°C for 75 minutes.

CF₃CCl₂CF₃ and H₂ were contacted with the catalyst at 350°C or 360°C and with a molar ratio of H₂:CF₃CCl₂CF₃ as shown in the table. Runs 1 to 3 were conducted at a pressure of 40 psig (380 kPa) and runs 4 to 9 were done at 100 psig (790 kPa). Results of the HDH reaction are shown in Table 1.

**TABLE 1**

| Run No. | HDH T(°C) | Molar Ratio H₂:216aa | CT Min. | % Conv. 216aa | %Sel. to | | | | |
|---|---|---|---|---|---|---|---|---|---|
| | | | | | 1225zc | 236fa | 245fa | 1215xc | 226da |
| 1 | 350 | 7 | 0.58 | 92.9 | 12.5 | 0.1 | 0.2 | 75.9 | 6.4 |
| 2 | 350 | 12 | 0.58 | 99.3 | 15.7 | 0.3 | 0.2 | 72.9 | 5.4 |
| 3 | 360 | 12 | 0.57 | 99.5 | 19.1 | 0.5 | 0.3 | 71.6 | 3.4 |
| 4 | 360 | 10 | 0.54 | 99.3 | 23.0 | 0.6 | 0.4 | 67.4 | 3.7 |
| 5 | 360 | 22 | 0.55 | 99.4 | 21.4 | 0.6 | 0.4 | 72.6 | 1.2 |
| 6 | 350 | 22 | 0.56 | 99.4 | 14.6 | 0.4 | 0.2 | 78.4 | 2.8 |
| 7 | 350 | 11 | 0.56 | 99.0 | 11.1 | 0.3 | 0.1 | 78.8 | 6.5 |
| 8 | 360 | 11 | 0.55 | 99.2 | 12.5 | 0.3 | 0.2 | 77.1 | 6.6 |
| 9 | 360 | 22 | 0.55 | 99.4 | 17.3 | 0.5 | 0.2 | 76.8 | 1.9 |

| | | | | | | | | | |
|---|---|---|---|---|---|---|---|---|---|
| ¹HDH is the hydrodehalogenation catalyst | | | | | | | | | |

### REFERENCE EXAMPLE 2

The reactor used for hydrodehalogenation (HDH) in Example 1 was charged with catalyst (24.1 g, 18.0 mL) 1/8" x 1/8" (3.2 mm x 3.2 mm) pellets prepared substantially in accordance with the Catalyst Preparation above. The HDH catalyst unlike that of Example 1 was not used previously, i.e., it was a fresh catalyst. Before starting the runs shown in Example 1. the HDH catalyst was treated with 110 sccm (1.8 x 10⁻⁶ m³/s) H₂ at 350°C and 40 psig (380 kPa) for one hour.

CF₃CCl₂CF₃ and H₂ were contacted with the catalyst at 350°C or 360°C and with a molar ratio of H₂:CF₃CCl₂CF₃ as shown in the table. All runs were conducted at a pressure of 40 psig (380 kPa). Results of the HDH reaction are shown in Table 2.

**TABLE 2**

| Run No. | HDH¹ T(°C) | Molar Ratio H₂:216aa | CT Min. | %Conv. 216aa | %Sel. to | | |
|---|---|---|---|---|---|---|---|
| | | | | | 1225zc | 1215xc | Other² |
| 1 | 350 | 16 | 0.27 | 100 | 9.1 | 87.4 | 3.6 |
| 2 | 350 | 16 | 0.27 | 100 | 10.2 | 86.2 | 3.7 |
| 3 | 350 | 8 | 0.27 | 100 | 12.0 | 84.0 | 4.0 |
| 4 | 350 | 16 | 0.27 | 100 | 10.9 | 84.8 | 4.2 |

| | | | | | | | |
|---|---|---|---|---|---|---|---|
| ¹HDH is the hydrodehalogenation catalyst ²Other includes 236fa, 245fa and 226da | | | | | | | |

## Claims

1. An azeotropic composition comprising CF₃CHFCF₃ and HF, wherein said HF is present in an amount effective to form an azeotropic combination with said CF₃CHFCF₃.

2. The azeotropic composition of claim 1 wherein said HF is present in an amount o from 29.9 to 41.3 mole percent.

3. The azeotropic composition of claim 1 which consists essentially of from 29.9 to 41.3 mole percent HF and from 70.1 to 58.7 mole percent CF₃CHFCF₃.

4. A process for recovering HF from a product mixture comprising HF and CF₃CHFCF₃, comprising:
(1) distilling the product mixture to remove all products which have a lower boiling point than the lowest boiling azeotrope containing HF and CF₃CHFCF₃;and
(2) distilling said azeotrope to recover HF as an azeotropic composition containing HF and CF₃CHFCF₃.

## Patentansprüche

1. Azeotrope Zusammensetzung umfassend CF₃CHFCF₃ und HF, wobei HF in einer Menge vorliegt, die wirksam ist, um eine azeotrope Kombination mit dem CF₃CHFCF₃ zu bilden.

2. Azeotrope Zusammensetzung nach Anspruch 1, wobei das HF in einer Menge von 29,9 bis 41,3 Molprozent vorliegt.

3. Azeotrope Zusammensetzung nach Anspruch 1, die im Wesentlichen aus 29,9 bis 41,3 Molprozent HF und 70,1 bis 58,7 Molprozent CF₃CHFCF₃ besteht.

4. Verfahren zum Gewinnen von HF aus einer Produktmischung umfassend HF und CF₃CHFCF₃, umfassend:
(1) das Destillieren der Produktmischung, um alle Produkte zu entfernen, die einen niedrigeren Siedepunkt aufweisen als der niedrigstsiedende, HF und CF₃CHFCF₃ enthaltende Azeotrop; und
(2) das Destillieren des Azeotrops, um HF als azeotrope, HF und CF₃CHFCF₃ enthaltende Zusammensetzung zu gewinnen.

## Revendications

1. Composition azéotropique comprenant CF₃CHFCF₃ et HF, dans laquelle ledit HF est présent en une quantité efficace pour former une combinaison azéotropique avec ledit CF₃CHFCF₃.

2. Composition azéotropique selon la revendication 1, dans laquelle ledit HF est présent en une quantité de 29,9 à 41,3 pour cent en moles.

3. Composition azéotropique selon la revendication 1, qui consiste essentiellement en 29,9 à 41,3 pour cent en moles de HF et de 70,1 à 58,7 pour cent en moles de CF₃CHFCF₃.

4. Procédé pour récupérer HF à partir d'un mélange de produits comprenant HF et CF₃CHFCF₃, comprenant:
(1) la distillation du mélange de produits pour éliminer tous les produits qui ont un point d'ébullition plus bas que l'azéotrope ayant le plus bas point d'ébullition contenant HF et CF₃CHFCF₃; et
(2) la distillation dudit azéotrope pour récupérer HF sous la forme d'une composition azéotropique contenant HF et CF₃CHFCF₃.
